# FASCICULE DE BREVET EUROPEEN

(11) **EP 1 237 501 B1**
(45) Date de publication et mention de la délivrance du brevet: **26.05.2004**
(21) Numéro de dépôt: 00954244.0
(22) Date de dépôt: 01.09.2000
(51) Int. Cl.: A61F 2/04, A61F 2/06

(54) **PROTHESE DESTINEE A CONTROLER LE SENS D'ECOULEMENT DANS UN CONDUIT D'UN ORGANISME VIVANT**
PROTHESE ZUM KONTROLLIEREN DER FLUSSRICHTUNG IN EINEM KÖRPERGEFÄSS
PROSTHESIS FOR CONTROLLING THE DIRECTION OF FLOW IN A DUCT OF A LIVING ORGANISM

(30) Priorité: 13.12.1999 EP 99811143
(43) Date de publication de la demande: 11.09.2002
(73) Titulaire: Biomedix S.A., 1701 Fribourg (CH)
(72) Inventeur: GODIN, Norman, CH-1208 Geneve (CH)
(74) Mandataire: Savoye, Jean-Paul
(86) Numéro de dépôt international: PCT/CH2000/000465
(87) Numéro de publication internationale: WO 2001/043663

(56) Documents cités:
- WO-A-91/01117
- WO-A-96/29954
- FR-A- 2 694 688
- US-A- 5 820 584

## Description

La présente invention se rapporte à une prothèse de forme générale tubulaire destinée à contrôler le sens de l'écoulement dans un conduit d'un organisme vivant, cette prothèse étant en un matériau élastiquement déformable biocompatible et comprenant une partie proximale annulaire de fixation dans une portion de plus grand diamètre dudit conduit, et une partie distale dont la paroi est dimensionnée pour lui permettre de s'écraser sur elle-même en présence d'une surpression exercée sur sa surface externe.

De telles prothèses ont notamment été proposées pour contrôler le reflux gastrique dans l'oesophage chez des personnes souffrant généralement d'une hernie hiatale accompagnée d'une oesophagite de reflux. Ces reflux répétés d'acide gastrique attaquent la paroi de l'oesophage et créent finalement une oesophagite qui se traduit par une ulcération de la paroi et parfois un rétrécissement de sa section de passage. On a proposé, notamment dans le WO 96/29954 une prothèse destinée à permettre l'écoulement des aliments de l'oesophage vers l'estomac mais d'arrêter le reflux du contenu gastrique de l'estomac dans l'oesophage aux pressions de reflux en se repliant et/ou se "collapsant".

Le problème que posent de telles prothèses est celui de leur fixation et celui de leur changement donc aussi bien de leur mise en place que de leur enlèvement. En effet, cette affection étant chronique, un malade devra être muni constamment d'une telle prothèse, de sorte que celle-ci devra être changée périodiquement, notamment en raison du vieillissement des matières utilisées, généralement des élastomères, et des conditions particulièrement agressives du milieu dans lequel elle se trouve, l'acide gastrique ayant un pH de l'ordre de 1. Or, les seules solutions proposées sont soit chirurgicales, soit des éléments d'accrochage dont la nature n'est pas spécifiée. La voie chirurgicale ne serait acceptable à la rigueur que si la prothèse pouvait être garantie pour la durée de vie du patient. Or, on sait bien que tout élastomère vieillit et que le milieu dans lequel la prothèse est appelée à fonctionner est en plus particulièrement agressif, de sorte que la voie chirurgicale ne constitue pas une solution puisqu'on devrait y avoir recours périodiquement, alors qu'il existe d'autres voies thérapeutiques médicamenteuses comprenant les antiacides qui tendent à rendre neutre le milieu, les anti-histaminiques H2 qui se fixent sur les récepteurs H2 de la cellule pariétale ainsi que des médicaments qui bloquent la production des ions H+ par la cellule pariétale. Ces médicaments ont en commun de ne pas guérir le mal dont souffre le patient, de sorte qu'ils doivent être administrés de façon continue. Il est évident qu'à ce jour, aucune des solutions proposées n'est satisfaisante, dans la mesure où elles ne proposent qu'une alternative entre l'administration continue de médicaments et des interventions chirurgicales répétées.

Il est évident que la prothèse constitue sans doute la meilleure solution potentielle, dans la mesure où elle permet de remédier par des moyens purement mécaniques au dysfonctionnement dont souffre le patient. Cependant cette solution n'est envisageable qu'à partir du moment où la pose et l'enlèvement de cette prothèse pourraient être réalisées par voie endoscopique.

C'est précisément le but de la présente invention de proposer une solution qui permette la pose aussi bien que l'enlèvement de la prothèse par voie endoscopique.

A cet effet, la présente invention a pour objet une prothèse de forme générale tubulaire selon la revendication

Le dessin annexé illustre, schématiquement et à titre d'exemple, deux formes d'exécution de la prothèse objet de la présente invention.
La figure 1 est une vue en élévation d'une prothèse selon cette forme d'exécution;
la figure 2 est une vue en coupe selon II-II de la figure 1;
la figure 3 est une vue en perspective d'un détail d'un élément de raidissement de l'extrémité proximale de la prothèse;
la figure 4 est une vue en perspective d'un autre élément de raidissement de l'extrémité distale de la prothèse à l'état contracté;
la figure 5 est une vue en perspective de l'élément illustré par la figure 4 à l'état non contracté,
la figure 6 est une vue en coupe de la base de l'oesophage et du haut de l'estomac avec l'extrémité proximale de la prothèse fixée dans une hernie hiatale,
la figure 7 est une vue en coupe semblable à la figure 6 d'une seconde forme d'exécution,
la figure 8 est une vue en perspective d'un élément de raidissement de la partie proximale de la prothèse,
la figure 9 est une vue en perspective d'une variante de l'élément de raidissement,
la figure 10 est une vue en perspective d'une deuxième variante de l'élément de raidissement,
la figure 11 est une vue en perspective coupée d'une prothèse comportant une autre variante encore de l'élément de raidissement.

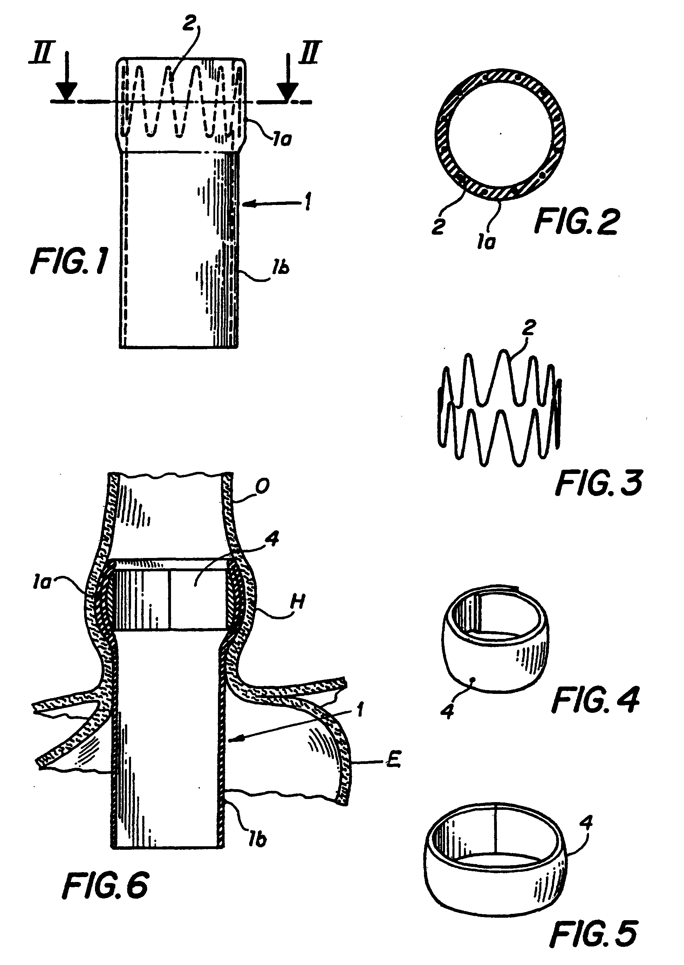

Sur la figure 1, une prothèse tubulaire 1 est représentée, venue d'une pièce par moulage avec une partie proximale annulaire de fixation la présentant une plus grande résistance à l'écrasement que la partie distale 1b. Cette prothèse 1 est destinée à empêcher le reflux oesophagien. Une telle prothèse est décrite en détail dans le WO 96/29954 auquel on pourra se référer pour plus de détails.

Cette prothèse tubulaire 1 est réalisée en un élastomère biocompatible, par exemple un élastomère à base de silicone à deux composants de qualité médicale, vendu sous la marque Silastic® par Dow Corning Corp, ou un silicone de la maison Nusil. On peut aussi utiliser des caoutchoucs de type butyle. Ces matériaux et leur épaisseur sont choisis pour qu'en présence d'une surpression exercée sur la face externe de la partie distale de la prothèse tubulaire 1, ses parois se rejoignent et empêchent ainsi le passage de substance de l'estomac vers l'oesophage.

Un élément élastique de renforcement ou de raidissement 2 est noyé dans la partie proximale annulaire 1a de la prothèse 1 pour former une partie annulaire de fixation. Cet élément élastique 2 doit exercer une force centrifuge destinée à appliquer l'extrémité proximale 1a de la prothèse 1 contre la paroi du conduit de l'organisme dans lequel elle doit prendre place, dans cet exemple, ce conduit étant la hernie hiatale que présentent la plupart des patients souffrant de reflux gastrique chronique, comme illustré par la figure 6. Cet élément élastique de renforcement 2 doit en même temps être suffisamment rétractable consécutivement à l'application d'une force centripète pour permettre de réduire très sensiblement sa section en vue de son insertion dans le conduit de l'organisme vivant par voie endoscopique ainsi que pour son retrait dudit conduit.

A cet effet, l'élément élastique de renforcement peut prendre différentes formes, telle que celle illustrée par la figure 3 dans laquelle l'élément élastique de renforcement est constitué par un fil à ressort 10, disposé en un anneau d'une certaine largeur formant des méandres réguliers d'un bord à l'autre de l'anneau. Cet élément élastique 2 peut être fabriqué par exemple en alliage à mémoire de forme en nickel-titane permettant une forte déformation lors de la pose et du retrait, ou en acier ressort de qualité médicale. Un élastomère dans lequel l'élément élastique 2 est noyé est biocompatible et protège la paroi des tissus de toute blessure due à l'élément élastique et empêche le déplacement de la prothèse.

L'élément élastique de renforcement ou de raidissement 2 peut être fortement déformé par une force centripète afin de réduire sa section en vue de lui permettre d'être introduit par voie endoscopique.

Comme illustré par la figure 6, la prothèse 1 est fixée entre la base de l'oesophage O et le haut de l'estomac E, en général dans une partie formée par une hernie hiatale H. La partie de la prothèse 1 utilisée pour sa fixation est la partie proximale la dont la résistance à l'écrasement est sensiblement plus élevé que celle du reste de l'élément tubulaire 1 et dans laquelle l'élément élastique de renforcement 2 est noyé. On voit sur cette figure 6 qu'une bague fendue 4 est disposée à l'intérieur de la partie proximale annulaire de fixation 1a.

Cette bague fendue 4 est une bague élastique, susceptible de s'enrouler en spirale, comme illustré par la figure 4, lorsqu'une pression centripète lui est appliquée. En la libérant, elle reprend sa section initiale comme illustré par la figure 5. Pour lui permettre d'épouser la forme de la hernie, sa face externe peut avantageusement être bombée, comme on le voit en particulier sur les figures 4 et 5. Il est évidemment également possible d'utiliser une lame-ressort d'égale épaisseur et de la cintrer dans sa largeur.

La bague élastique fendue 4 permet, en choisissant une dimension appropriée au diamètre de la hernie hiatale H, d'exercer une pression centrifuge sur la partie annulaire de fixation la de la prothèse 1, apte à maintenir cette prothèse en place avec suffisamment de force pour assurer son maintien en résistant au passage du bol alimentaire à travers elle. Le matériau utilisé pour réaliser la bague élastique peut être un métal, tel que de l'acier inoxydable, ou une matière plastique suffisamment rigide et élastique, ou encore un métal revêtu d'une matière plastique biocompatible.

La pose de la prothèse 1 utilisant la bague élastique fendue 4 s'effectue en deux temps, entièrement par voie endoscopique. Dans un premier temps, la prothèse souple 1 est repliée sur elle-même pour réduire son diamètre et permettre de l'introduire à travers l'oesophage O à l'aide d'un endoscope (non représenté), en positionnant la partie de fixation 1a dans l'hernie hiatale H.

Une fois la prothèse 1 positionnée, on exerce une force centripète sur la bague fendue 4, par exemple à l'aide d'une pince ou d'un organe d'enroulement du type utilisé par les horlogers pour mettre un ressort-spiral dans un barillet (non représenté). On descend la bague élastique fendue 4 à travers l'oesophage O jusqu'à la position désirée et on libère cette bague 4, qui tend à reprendre sa section initiale sous l'effet de son élasticité.

Grâce à la possibilité d'enrouler la bague élastique fendue 4 en spirale pour réduire son diamètre, la force susceptible d'être développée par une telle bague fendue 4 est supérieure à celle du fil métallique 2 noyé dans la partie de fixation annulaire 1a de la prothèse 1. Les dimensions de la bague fendue 4 peuvent être choisies, d'une part en fonction des dimensions de la hernie hiatale, d'autre part en fonction de la force que l'on désire obtenir en position de fixation de la prothèse 1 dans la hernie H.

Dans une variante non représentée, la bague élastique fendue 4 pourrait être logée dans un espace annulaire ménagé à l'intérieur de la partie proximale annulaire de fixation 1a et remplacer ainsi l'élément élastique de renforcement 2 noyé dans cette partie de fixation annulaire 1a. Dans ce cas, il faut que l'espace annulaire destiné à recevoir la bague élastique fendue 4 permette l'enroulement de cette bague en spirale lorsqu'une pression centripète est appliquée à la partie de fixation annulaire 1a.

A cet effet, une extrémité de cet espace annulaire peut être fermée, tandis que l'autre extrémité s'ouvre sur la face interne de la partie proximale annulaire de fixation 1a. Grâce à cette conformation de l'espace annulaire, lorsqu'une pression centripète s'exerce sur cette partie proximale annulaire de fixation 1a, une extrémité de la bague élastique fendue 4 bute contre l'extrémité fermée de l'espace annulaire, de sorte que son autre extrémité sort par l'extrémité de cet espace s'ouvrant sur la face interne de la partie de fixation annulaire la permettant de réduire le diamètre de la partie annulaire de fixation 1a de la prothèse 1. En relâchant la pression centripète, la bague fendue 4 se dilate pour tendre à reprendre son diamètre initial. Avantageusement, l'extrémité de la bague fendue, adjacente à l'extrémité fermée de l'espace annulaire ménagé dans la partie proximale annulaire de fixation, pourrait être fixée à cette extrémité.

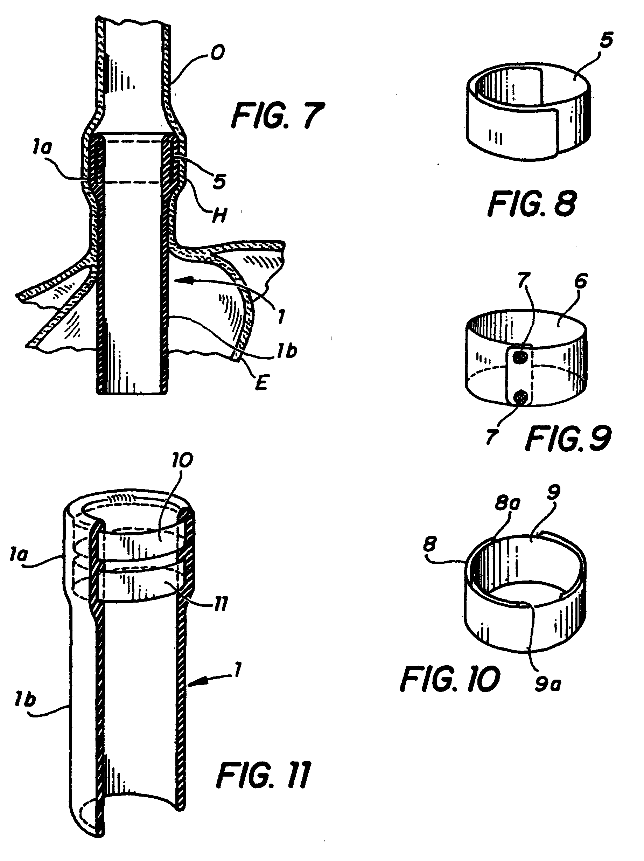

La seconde forme d'exécution et ses variantes illustrées par les figures 7 à 11 se rapporte plus spécialement à l'utilisation d'alliage à mémoire de forme destinés à raidir l'extrémité proximale 1a de la prothèse alors que le reste de cette prothèse allant jusqu'à l'extrémité distale du corps tubulaire 1 reste suffisamment souple pour que ses parois s'écrasent en présence d'une surpression provoquée par un reflux gastrique. Dans cette forme d'exécution, on n'utilise pratiquement pas l'élasticité du matériau, mais ses deux états: mou permettant une déformation plastique du matériau, relativement rigide tout en permettant de retrouver sa forme initiale.

L'élément de raidissement en alliage à mémoire de forme 5 illustré par les figures 7 et 8 est constitué par une lame enroulée en spirale. Contrairement à la première forme d'exécution, ce n'est donc pas l'élasticité de cet élément 5 qui va être utilisé, mais sa capacité à se déformer de manière plastique lorsque l'on abaisse sa température suffisamment bas, au-dessous de la température du corps, pour se transformer en phase martensite dans laquelle il est relativement mou et sa capacité à reprendre sa forme initiale et à se transformer en phase austénite relativement rigide lorsque l'on élève sa température au-dessus de la température du corps humain, dans le cas présent au-dessus de 45°C pour un alliage 55,68% en poids de Ni, Ti pour le reste, en l'absence de contrainte.

Ces conditions sont tout à fait acceptables pour l'application envisagée. En effet, le diamètre de la partie proximale la de la prothèse 1 doit être dimensionné initialement pour s'adapter au diamètre de la hernie hiatale H et le diamètre initial de l'élément de raidissement 5 en alliage à mémoire de forme est dimensionné pour épouser ce diamètre lorsqu'il est porté à la température de transformation en phase austénite où il est en même temps le plus rigide, de sorte qu'il permet à l'extrémité proximale la de la prothèse 1 de s'adapter à la hernie et de l'empêcher d'en ressortir.

Pour obtenir ce résultat, l'élément de raidissement 5 doit initialement être refroidi au-dessous de la température du corps humain pour le transformer en phase martensite pour qu'il devienne relativement mou. Dans cet état il peut être replié sur lui-même ou déformé d'une autre manière pour l'introduire dans l'oesophage à l'aide d'un endoscope. Une fois positionné dans la hernie hiatale H, il suffit d'envoyer de l'eau à une température convenable ou un autre moyen de chauffage convenable, pour chauffer l'alliage par exemple à 60°C pendant une durée appropriée, pour effectuer la transformation de l'alliage à mémoire de forme dans sa phase austénite et pour lui faire simultanément retrouver sa forme initiale dans laquelle il est appliqué contre la paroi de la hernie hiatale H. En effet, comme l'élément de raidissement 5 en alliage à mémoire de forme est initialement dimensionné au diamètre de la hernie hiatale, aucune contrainte n'est exercée sur lui de sorte qu'il peut retrouver sa forme initiale.

Pour améliorer le raidissement conféré à l'extrémité proximale de la prothèse 1, on pourrait aussi envisager de former un anneau 6 dont les deux extrémités sont fixées l'une à l'autre par soudage ou collage 7, comme illustré par la figure 9. On peut aussi utiliser deux anneaux ouverts 8, 9 disposés l'un à l'intérieur de l'autre en décalant les ouvertures respectives 8a, 9a de ces anneaux ouverts 8, 9 de 180° l'une de l'autre. Enfin, comme illustré par la figure 11, on peut encore envisager de décaler axialement deux anneaux en alliage à mémoire de forme 10, 11, l'espace annulaire entre ces anneaux pouvant servir à poser quelques points de suture par exemple.

## Revendications

1. Prothèse de forme générale tubulaire (1) destinée à contrôler le sens de l'écoulement du bol alimentaire dans le conduit oesophagien (O) d'un organisme vivant, cette prothèse (1) étant en un matériau élastiquement déformable biocompatible et comprenant une partie proximale annulaire de fixation (1a) dans une portion (H) de plus grand diamètre dudit conduit (O), et une partie distale (1b) dont la paroi est dimensionnée pour lui permettre de s'écraser sur elle-même en présence d'une surpression exercée sur sa surface externe, dans laquelle ladite partie proximale annulaire de fixation (1a) est associée sur une certaine largeur à des moyens de raidissement pour lui conférer une résistance à l'écrasement plus élevée que celle de la partie distale (1b) de ladite prothèse (1), apte à retenir la prothèse en appliquant ladite partie proximale annulaire (1a) contre la paroi de ladite portion (H) de plus grand diamètre dudit conduit (O) avec une force suffisante pour assurer son maintien au passage du bol alimentaire à travers elle, **caractérisée par le fait que** ladite résistance à l'écrasement plus élevée de ladite partie proximale annulaire est obtenue par des moyens élastiques annulaires associés à cette partie proximale, lesdits moyens élastiques annulaires étant constitués au moins partiellement par une bague élastique fendue (4).

2. Prothèse selon la revendication 1, **caractérisée en ce que** ladite bague fendue (4) est un élément indépendant de ladite prothèse rapporté à l'intérieur de ladite partie proximale annulaire (1a).

3. Prothèse de forme générale tubulaire (1) destinée à contrôler le sens de l'écoulement du bol alimentaire dans le conduit oesophagien (O) d'un organisme vivant, cette prothèse (1) étant en un matériau élastiquement déformable biocompatible et comprenant une partie proximale annulaire de fixation (1a) dans une portion (H) de plus grand diamètre dudit conduit (O), et une partie distale (1b) dont la paroi est dimensionnée pour lui permettre de s'écraser sur elle-même en présence d'une surpression exercée sur sa surface externe, dans laquelle ladite partie proximale annulaire de fixation (1a) est associée sur une certaine largeur à des moyens de raidissement pour lui conférer une résistance à l'écrasement plus élevée que celle de la partie distale (1b) de ladite prothèse (1), apte à retenir la prothèse en appliquant ladite partie proximale annulaire (1a) contre la paroi de ladite portion (H) de plus grand diamètre dudit conduit (O) avec une force suffisante pour assurer son maintien au passage du bol alimentaire à travers elle, ladite prothèse (1) étant en élastomère, **caractérisée par le fait que** la dite résistance à l'écrasement plus élevée de la dite partie proximale annulaire est obtenue par des moyens élastiques annulaires, lesdits moyens élastiques annulaires étant constitués par au moins un ressort (2) noyé dans l'élastomère de ladite prothèse (1).

4. Prothèse selon la revendication 3, **caractérisée par le fait que** ledit ressort (2) est un fil sans fin, formant une série de méandres réguliers s'étendant d'un bord à l'autre d'un anneau.

5. Prothèse de forme générale tubulaire (1) destinée à contrôler le sens de l'écoulement du bol alimentaire dans le conduit oesophagien (O) d'un organisme vivant, cette prothèse (1) étant en un matériau élastiquement déformable biocompatible et comprenant une partie proximale annulaire de fixation (1a) dans une portion (H) de plus grand diamètre dudit conduit (O), et une partie distale (1b) dont la paroi est dimensionnée pour lui permettre de s'écraser sur elle-même en présence d'une surpression exercée sur sa surface externe, dans laquelle ladite partie proximale annulaire de fixation (1a) est associée sur une certaine largeur à des moyens de raidissement pour lui conférer une résistance à l'écrasement plus élevée que celle de la partie distale (1b) de ladite prothèse (1), apte à retenir la prothèse en appliquant ladite partie proximale annulaire (1a) contre la paroi de ladite portion (H) de plus grand diamètre dudit conduit (O) avec une force suffisante pour assurer son maintien au passage du bol alimentaire à travers elle, **caractérisée par le fait que** ladite partie proximale annulaire (1a) renferme un logement annulaire dont une extrémité est fermée et l'autre est ouverte sur la face interne de ladite partie proximale annulaire (1a) ce dit logement annulaire recevant une bague élastique fendue (4).

6. Prothèse de forme générale tubulaire (1) destinée à contrôler le sens de l'écoulement du bol alimentaire dans le conduit oesophagien (O) d'un organisme vivant, cette prothèse (1) étant en un matériau élastiquement déformable biocompatible et comprenant une partie proximale annulaire de fixation (1a) dans une portion (H) de plus grand diamètre dudit conduit (O), et une partie distale (1b) dont la paroi est dimensionnée pour lui permettre de s'écraser sur elle-même en présence d'une surpression exercée sur sa surface externe, dans laquelle ladite partie proximale annulaire de fixation (1a) est associée sur une certaine largeur à des moyens de raidissement pour lui conférer une résistance à l'écrasement plus élevée que celle de la partie distale (1b) de ladite prothèse (1), apte à retenir la prothèse en appliquant ladite partie proximale annulaire (1a) contre la paroi de ladite portion (H) de plus grand diamètre dudit conduit (O) avec une force suffisante pour assurer son maintien au passage du bol alimentaire à travers elle, **caractérisée par le fait que** ladite résistance à l'écrasement plus élevée de ladite partie proximale annulaire est obtenue par au moins un élément (5) réalisé en un alliage à mémoire de forme associé à ladite partie proximale annulaire.

7. Prothèse selon la revendication 6, **caractérisée en ce que** ladite résistance à l'écrasement plus élevée de ladite partie proximale annulaire est obtenue par deux bagues fendues concentriques et coplanaires en alliage à mémoire de forme.

8. Prothèse selon la revendication 6, **caractérisée en ce que** ladite résistance à l'écrasement plus élevée de ladite partie proximale annulaire est obtenue par deux bagues fendues coaxiales en alliage à mémoire de forme, séparées axialement l'une par rapport à l'autre.

9. Prothèse selon l'une des revendications 6 à 8, **caractérisée en ce que** la plage de température de transformation martensitique dudit alliage à mémoire de forme se situe sensiblement au-dessous de la température du corps humain, tandis que sa température de fin de transformation austénitique se situe sensiblement au-dessus de la température du corps humain.

## Patentansprüche

1. Prothese von allgemein röhrenförmiger Gestalt (1), dazu bestimmt, die Flussrichtung des Speisebreis in der Speiseröhre (O) eines lebenden Organismus zu kontrollieren, wobei diese Prothese (1) aus einem biokompatiblen, elastisch verformbaren Material besteht und einen proximalen, ringförmigen Befestigungsabschnitt (1a) in einem Teil (H) grösseren Durchmessers der Speiseröhre (O) sowie einen distalen Teil (1b) umfasst, dessen Wandung so bemessen ist, dass sie in Gegenwart eines auf ihre Aussenseite ausgeübten Überdrucks zusammenfällt, und in der dem proximalen, ringförmigen Befestigungsabschnitt (1a) über eine bestimmte Länge Versteifungsmittel beigefügt sind, um ihm eine höhere Druckfestigkeit als die des distalen Abschnittes (1b) der Prothese (1) zu erteilen, und er in der Lage ist, die Prothese zurückzuhalten, indem dieser proximale, ringförmige Abschnitt (1a) mit einer Kraft, die genügt, um seinen Halt beim Durchgang des Speisebreis zu bewahren, gegen die Wandung des Teils (H) grösseren Durchmessers der Speiseröhre (O) gedrückt wird, **dadurch gekennzeichnet, dass** die höhere Druckfestigkeit des proximalen, ringförmigen Abschnitts durch ringförmige, elastische Organe erhalten wird, die diesem proximalen Abschnitt beigefügt sind, wobei diese ringförmigen, elastischen Organe zumindest teilweise aus einem elastischen Schlitzring (4) bestehen.

2. Prothese nach Anspruch 1, **dadurch gekennzeichnet, dass** der Schlitzring (4) ein Element ist, das von der Prothese unabhängig ist und am Inneren des ringförmigen, proximalen Abschnitts (1a) angebracht ist.

3. Prothese von allgemein röhrenförmiger Gestalt (1), dazu bestimmt, die Flussrichtung des Speisebreis in der Speiseröhre (O) eines lebenden Organismus zu kontrollieren, wobei diese Prothese (1) aus einem biokompatiblen, elastisch verformbaren Material besteht und einen proximalen, ringförmigen Befestigungsabschnitt (1a) in einem Teil (H) grösseren Durchmessers der Speiseröhre (O) sowie einen distalen Teil (1b) umfasst, dessen Wandung so bemessen ist, dass sie in Gegenwart eines auf ihre Aussenseite ausgeübten Überdrucks zusammenfällt, und in der dem proximalen, ringförmigen Befestigungsabschnitt (1a) über eine bestimmte Länge Versteifungsmittel beigefügt sind, um ihm eine höhere Druckfestigkeit als die des distalen Abschnittes (1b) der Prothese (1) zu erteilen, und er in der Lage ist, die Prothese zurückzuhalten, indem dieser proximale, ringförmige Abschnitt (1a) mit einer Kraft, die genügt, um seinen Halt beim Durchgang des Speisebreis zu bewahren, gegen die Wandung des Teils (H) grösseren Durchmessers der Speiseröhre (O) gedrückt wird, **dadurch gekennzeichnet, dass** die höhere Druckfestigkeit des proximalen, ringförmigen Abschnitts durch ringförmige, elastische Organe erhalten wird, wobei die ringförmigen, elastischen Organe aus zumindest einer Feder (2) bestehen, die in das Elastomer dieser Prothese (1) eingelassen ist.

4. Prothese nach Anspruch 3, **dadurch gekennzeichnet, dass** die Feder (2) ein Endlosdraht ist, der eine Reihe regelmässiger Mäander bildet, die sich von einem Rand eines Ringes zum anderen erstrecken.

5. Prothese von allgemein röhrenförmiger Gestalt (1), dazu bestimmt, die Flussrichtung des Speisebreis in der Speiseröhre (O) eines lebenden Organismus zu kontrollieren, wobei diese Prothese (1) aus einem biokompatiblen, elastisch verformbaren Material besteht und einen proximalen, ringförmigen Befestigungsabschnitt (1a) in einem Teil (H) grösseren Durchmessers der Speiseröhre (O) sowie einen distalen Teil (1b) umfasst, dessen Wandung so bemessen ist, dass sie in Gegenwart eines auf ihre Aussenseite ausgeübten Überdrucks zusammenfällt, und in der dem proximalen, ringförmigen Befestigungsabschnitt (1a) über eine bestimmte Länge Versteifungsmittel beigefügt sind, um ihm eine höhere Druckfestigkeit als die des distalen Abschnittes (1b) der Prothese (1) zu erteilen, und er in der Lage ist, die Prothese zurückzuhalten, indem dieser proximale, ringförmige Abschnitt (1a) mit einer Kraft, die genügt, um seinen Halt beim Durchgang des Speisebreis zu bewahren, gegen die Wandung des Teils (H) grösseren Durchmessers der Speiseröhre (O) gedrückt wird, **dadurch gekennzeichnet, dass** der proximale, ringförmige Abschnitt (1a) ein ringförmiges Lager umschliesst, dessen eines Ende verschlossen ist, während das andere Ende zur Innenseite des proximalen, ringförmigen Abschnitts (1a) hin offen ist, wobei dieses ringförmige Lager einen elastischen Schlitzring (4) aufnimmt..

6. Prothese von allgemein röhrenförmiger Gestalt (1), dazu bestimmt, die Flussrichtung des Speisebreis in der Speiseröhre (O) eines lebenden Organismus zu kontrollieren, wobei diese Prothese (1) aus einem biokompatiblen, elastisch verformbaren Material besteht und einen proximalen, ringförmigen Befestigungsabschnitt (1a) in einem Teil (H) grösseren Durchmessers der Speiseröhre (O) sowie einen distalen Teil (1b) umfasst, dessen Wandung so bemessen ist, dass sie in Gegenwart eines auf ihre Aussenseite ausgeübten Überdrucks zusammenfällt, und in der dem proximalen, ringförmigen Befestigungsabschnitt (1a) über eine bestimmte Länge Versteifungsmittel beigefügt sind, um ihm eine höhere Druckfestigkeit als die des distalen Abschnittes (1b) der Prothese (1) zu erteilen, und er in der Lage ist, die Prothese zurückzuhalten, indem dieser proximale, ringförmige Abschnitt (1a) mit einer Kraft, die genügt, um seinen Halt beim Durchgang des Speisebreis zu bewahren, gegen die Wandung des Teils (H) grösseren Durchmessers der Speiseröhre (O) gedrückt wird, **dadurch gekennzeichnet, dass** die höhere Druckfestigkeit des proximalen, ringförmigen Abschnitts durch zumindest ein Element (5) erhalten wird, das aus einer Formgedächtnislegierung besteht und dem proximalen, ringförmigen Abschnitt beigefügt ist.

7. Prothese nach Anspruch 6, **dadurch gekennzeichnet, dass** die höhere Druckfestigkeit des proximalen, ringförmigen Abschnitts durch zwei konzentrische und koplanare Schlitzringe aus Formgedächtnislegierung erhalten wird.

8. Prothese nach Anspruch 6, **dadurch gekennzeichnet, dass** die höhere Druckfestigkeit des proximalen, ringförmigen Abschnitts durch zwei koaxiale Schlitzringe aus Formgedächtnislegierung erhalten wird, die axial voneinander beabstandet sind.

9. Prothese nach einem der Ansprüche 6 bis 8, **dadurch gekennzeichnet, dass** der Temperaturbereich der martensitischen Umwandlung der Formgedächtnislegierung merklich unter der menschlichen Körpertemperatur liegt, während ihre Endtemperatur der austenitischen Umwandlung merklich über der menschlichen Körpertemperatur liegt.

## Claims

1. Prosthesis (1) of generally tubular shape for controlling the direction of flow of the food bolus in the oesophageal duct (O) of a living organism, this prosthesis (1) being made of a biocompatible, elastically deformable material and comprising an annular proximal fixing part (1a) in a section (H) of greater diameter of said duct (O), and a distal part (1b) whose wall is dimensioned to allow it to collapse on itself in the presence of an overpressure exerted on its outer surface, in which prosthesis said annular proximal fixing part (1a) is associated over a certain width with stiffening means for giving it a resistance to crushing which is greater than that of the distal part (1b) of said prosthesis (1), able to retain the prosthesis by applying said annular proximal part (1a) against the wall of said section (H) of greater diameter of said duct (O) with a force sufficient to ensure that it is held in place as the food bolus passes through it, **characterized in that** said greater resistance to crushing of said annular proximal part is obtained by annular elastic means associated with this proximal part, said annular elastic means being formed at least partially by a slotted elastic ring (4).

2. Prosthesis according to Claim 1, **characterized in that** said slotted ring (4) is an independent element of said prosthesis and is attached inside said annular proximal part (1a).

3. Prosthesis (1) of generally tubular shape for controlling the direction of flow of the food bolus in the oesophageal duct (O) of a living organism, this prosthesis (1) being made of a biocompatible, elastically deformable material and comprising an annular proximal fixing part (1a) in a section (H) of greater diameter of said duct (O), and a distal part (1b) whose wall is dimensioned to allow it to collapse on itself in the presence of an overpressure exerted on its outer surface, in which prosthesis said annular proximal fixing part (1a) is associated over a certain width with stiffening means for giving it a resistance to crushing which is greater than that of the distal part (1b) of said prosthesis (1), able to retain the prosthesis by applying said annular proximal part (1a) against the wall of said section (H) of greater diameter of said duct (O) with a force sufficient to ensure that it is held in place as the food bolus passes through it, said prosthesis (1) being made of elastomer, **characterized in that** said greater resistance to crushing of said annular proximal part is obtained by annular elastic means, said annular elastic means consisting of at least one spring (2) embedded in the elastomer of said prosthesis (1).

4. Prosthesis according to Claim 3, **characterized in that** said spring (2) is an endless filament forming a series of regular meanders extending from one edge to the other of a ring.

5. Prosthesis (1) of generally tubular shape for controlling the direction of flow of the food bolus in the oesophageal duct (O) of a living organism, this prosthesis (1) being made of a biocompatible, elastically deformable material and comprising an annular proximal fixing part (1a) in a section (H) of greater diameter of said duct (O), and a distal part (1b) whose wall is dimensioned to allow it to collapse on itself in the presence of an overpressure exerted on its outer surface, in which prosthesis said annular proximal fixing part (1a) is associated over a certain width with stiffening means for giving it a resistance to crushing which is greater than that of the distal part (1b) of said prosthesis (1), able to retain the prosthesis by applying said annular proximal part (1a) against the wall of said section (H) of greater diameter of said duct (O) with a force sufficient to ensure that it is held in place as the food bolus passes through it, **characterized in that** said annular proximal part (1a) encloses an annular seat, one end of which is closed, and the other end of which is open on the inner face of said annular proximal part (1a), said annular seat receiving a slotted elastic ring (4).

6. Prosthesis (1) of generally tubular shape for controlling the direction of flow of the food bolus in the oesophageal duct (O) of a living organism, this prosthesis (1) being made of a biocompatible, elastically deformable material and comprising an annular proximal fixing part (1a) in a section (H) of greater diameter of said duct (O), and a distal part (1b) whose wall is dimensioned to allow it to collapse on itself in the presence of an overpressure exerted on its outer surface, in which prosthesis said annular proximal fixing part (1a) is associated over a certain width with stiffening means for giving it a resistance to crushing which is greater than that of the distal part (1b) of said prosthesis (1), able to retain the prosthesis by applying said annular proximal part (1a) against the wall of said section (H) of greater diameter of said duct (O) with a force sufficient to ensure that it is held in place as the food bolus passes through it, **characterized in that** said greater resistance to crushing of said annular proximal part is obtained by at least one element (5) made of a shape-memory alloy associated with said annular proximal part.

7. Prosthesis according to Claim 6, **characterized in that** said greater resistance to crushing of said annular proximal part is obtained by two concentric and coplanar slotted rings made of shape-memory alloy.

8. Prosthesis according to Claim 6, **characterized in that** said greater resistance to crushing of said annular proximal part is obtained by two coaxial slotted rings made of shape-memory alloy and separated axially from one another.

9. Prosthesis according to one of Claims 6 to 8, **characterized in that** the temperature range of martensitic transformation of said shape-memory alloy is situated substantially below the temperature of the human body, whereas its temperature at the end of austenitic transformation is situated substantially above the temperature of the human body.
